(19) Europäisches Patentamt
European Patent Office
Office européen des brevets

(11) Publication number: 0 076 430 B1

# (12) EUROPEAN PATENT SPECIFICATION

(45) Date of publication of patent specification: 18.06.86

(51) Int. Cl.$^4$: A 61 K 31/155

(21) Application number: 82108759.0

(22) Date of filing: 22.09.82

## (54) Compositions having antimicrobial activity.

(30) Priority: 05.10.81 IT 2431881

(43) Date of publication of application:
13.04.83 Bulletin 83/15

(45) Publication of the grant of the patent:
18.06.86 Bulletin 86/25

(84) Designated Contracting States:
BE CH DE FR GB IT LI LU NL SE

(56) References cited:
GB-A-2 042 528

(73) Proprietor: MEDEA RESEARCH S.r.l.
Via Cappuccini, 20
I-20100 Milano (IT)

(72) Inventor: Quadro, Giuseppe
Via Pisacane, 34/A
I-20100 Milan (IT)

(74) Representative: Bianchetti, Giuseppe
Studio Consulenza Brevettuale Via Rossini, 8
I-20122 Milan (IT)

EP 0 076 430 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to compositions endowed with local antiseptic activity. The compositions of the invention are characterized in that they contain, as active principle, an N-haloamidine of formula (1)

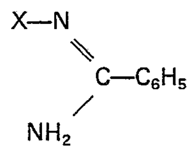

(1)

wherein X is a chlorine or a iodine atom.

Compounds of formula (1), per se known, and their preparation methods, are described in:

(1) P. Robin, C. R. Acad. Sci., Paris, *177*, 1304 (1923); and

(2) L. Citerio, D. Pocar, R. Stradi, B. Gioia, J. Chem. Soc. Perkin I, 1978, 509.

In Table 1 the formula of the active compounds, the corresponding abbreviations used in their biological screening, the molecular weight, the melting point and the method used particularly for their preparation are shown.

TABLE 1

| Compounds | Structural formula | M.P. | M.W. | Preparation's method |
|---|---|---|---|---|
| $AB_5$ | 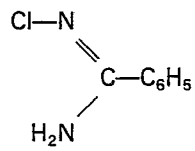 | 70—72 | 154,8 | (1) |
| $AB_6$ | 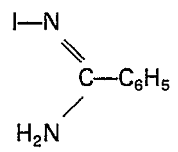 | 115—117 | 246 | (2) |

It is known that some N-halogen derivatives have antimicrobial activity (see for example G. Ehrhard and H. Ruschig, Arzneimittel, Vol. 4, pages 20 and following, Verlag Chemie, 1972). Such an activity, mainly due to the features of the nitrogen-halogen bond, derives also from the nature of the residual part of the compound. Nevertheless, the already known compounds, besides having sometimes weak activity, are often unstable and this limits the possibilities of practical use.

It has now been found that the compounds of formula (1) are endowed with marked local antiseptic activity and that, surprisingly, such an activity is considerably higher than that of active compounds containing N-halogen bonds, up to now used for this purpose.

As it will be evident from the data hereinafter reported, the compounds of the invention have partially a local antiseptic activity higher than that of the known antimicrobial agent chlorhexidine (N,N' - bis - (4 - chlorophenyl) - 3,12 - diimino - 2,4,11,13 - tetraazatetradecandiimidamide).

It is particularly interesting the activity of compounds $AB_5$ and $AB_6$ against strains of *Pseudomonas aeruginosa* and indole-positive *Proteus*, usually the most drug-resistant species.

The activity against these strains, has been clearly higher than that of chlorhexidine.

The bactericidal activity appears at the concentration of 100 p.p.m. in a different way on single strains, the bactericidal activity appearing at different contact times.

The particular action of compound $AB_6$ on *Pseudomonas* and indole-positive *Proteus* within 3 minutes (100% activity) and the action of compounds $AB_5$ on the same strains within 60—120 minutes, is emphasised.

The chlorhexidine, at the minimum bactericide concentration of 200 p.p.m., has proved to be less active. The serum has an inhibitory action on the activity (Tab. 4).

In conclusion, the main antibacterial interest of the investigated compounds of the invention is oriented on strains particularly resistant to drugs and disinfectant agents (*Pseudomonas aeruginosa* and indole-positive *Proteus*).

Taking into account the activities characterizing the cited compounds, the invention refers also to the use of the compounds of general formula (1) as local antiseptic agents. According to the invention, the meaning of "use" includes all the operations concerning the preparations of compounds of formula (1),

their purification, their formulation in forms suitable for their employment and/or for their making up in containers suitable for the employment to which they are designed.

For the practical use, the compounds of the present invention, corresponding to the formula (1), are appropriately transformed into suitable forms for the application to the areas and to the objects to be disinfected such as solutions, emulsions, suspensions, sprays, compositions in form of paste or powder.

For the preparation of such forms, the compounds of the invention are mixed up with suitable solvents, diluents or inert excipients.

The results of the studies which show the activity of the claimed compounds are hereinafter reported.

Materials and methods

Bacterial cultures

Different bacterial species isolated from patients are employed. When more strains of the same microorganism were used, these showed different antibiogram spectrum.

Strains of *E. coli*, *Pseudomonas aeruginosa*, indole-positive and negative *Proteus, Staphilococcus aureus, Enterobacter, Salmonella* sp., *Serratia marcescens, B. subtilis* and *Bacillaceae* are investigated.

Compounds $AB_5$ and $AB_6$ are dissolved in ethanol (ethyl alcohol 95°, C. Erba) and all the subsequent dilutions performed in distilled water. The chlorhexidine is diluted from the commercial product already dissolved in distilled water.

Determination of the minimum inhibitory concentration (MIC)

The broth dilution is employed to assess MIC values for all strains. Sterile microtiter plates with U bottom with 96 wells are employed. 0.1 Ml of liquid medium (tryptose Roche broth) are put in each well, the plates are kept at −20° and, before use, at room temperature. The disinfectant agent is put in the wells of the row 2 at a concentration of 4000 mcg/ml (real 2000 mcg/ml) and then with a 100 µl micropipette dilutions are performed from row 2 to row 11.

The row 1 is the control of the medium and the row 12 is the control for the fertility of the medium itself.

After having performed the dilutions, 0.01 ml of a 24 hrs bacterial broth culture (previously diluted at $10^{-4}$ for Gram-negative strains and $10^{-2}$ for Gram-positive ones) are placed in all wells, from the rows 2 to 12.

After the inoculation, the plate is hermetically sealed with adhesive transparent band and a hole is made with a needle.

The plates are kept at 35° for 24 hours and a macroscopic reading is carried out; in case of non-uniform reading, taking place only for chlorhexidine, the cultures from the wells are plated on agar-heart-brain medium (Sclavo) through Dinatech apparatus (head "Inoculum 96") and the reading of the growth is carried out in the following 24 hours.

Bactericide activity

It is assessed through Dinatech apparatus with an "Inoculum 96" head.

Chemical compounds at established concentrations are placed in amount of 1 ml in the containers, the bacterial inoculum is then added in amount of 0.1 ml (24 hrs culture) and at pre-established times (3,5,10,30,60 and 120 minutes), through the head, the mixture compound/disinfectant agent is transferred in solid medium (agar-heart-brain medium). After incubation at 35°C for 24 hours, the reading is carried out. The tests are repeated in presence of human serum at the concentration of 10%.

Results

The evaluation of the MIC, reported in Table 2, shows a higher value for chemical compounds $AB_5$ and $AB_6$ for *Proteus* and *Pseudomonas aeruginosa* strains.

Such an activity is higher than that showed by chlorhexidine.

Also the other compounds have higher antimicrobial activity for *Staphilcoccus aureus* and *Bacillaceae* in comparison with Gram-negative strains.

The bactericidal activity on the strains is reported in Table 3. The reported concentration of 100 p.p.m. for each chemical compound is the minimum one which proved to be active; in preliminary tests, concentrations of 10 and 1 p.p.m. proved to be in fact inactive. For chlorhexidine, the minimum active concentration is 200 p.p.m.

From the reported data on different strains, it comes out for *E. coli* total bactericide activity developing within 3 minutes for compound $AB_5$; activity noticed within 30—120 minutes for chlorhexidine. For *Pseudomonas aeruginosa* and indole-positive *Proteus* the total bactericide activity is within 3 minutes for compound $AB_6$, from 60 to 120 minutes for the chlorhexidine.

Both chemical compounds show activity on *Staph. aureus, B. subtilis, Serratia marcescens* and *Salmonella* sp.

Chemical compound $AB_6$ is the more active; the longest time in which it is active is 30 minutes for *Serratia marcescens.*

TABLE 2
Minimum inhibitory concentration of compounds $AB_5$ and $AB_6$ in comparison with chlorhexidine

| Microorganism | | Compounds Minimum inhibitor concentration (µg/ml) | | |
|---|---|---|---|---|
| | | $AB_5$ | $AB_6$ | Chlorhexidine |
| *E. coli* | 2094 | 250 | >1000 | — |
| | 2096 | 250 | >1000 | 1000 |
| | 2117 | 250 | >1000 | 1000 |
| | 2456 | 500 | >1000 | 500 |
| | 2568 | 500 | 2000 | 2000 |
| Indole- | 2083 | 125 | >1000 | 1000 |
| positive | 2105 | 500 | >1000 | >1000 |
| | 2075 | 250 | >1000 | >1000 |
| *Proteus* | 2410 | 250 | >2000 | 2000 |
| | 2447 | 500 | >2000 | 2000 |
| | 2674 | 500 | 1000 | >2000 |
| *Pseudomonas* | 2084 | 250 | >1000 | — |
| | 2458 | 500 | >1000 | 1000 |
| *aeruginosa* | 2509 | 250 | 1000 | 2000 |
| | 2488 | 250 | 1000 | 2000 |
| | 2670 | 500 | 1000 | >2000 |
| | 2508 | 500 | 1000 | >2000 |
| *Staph. aureus* | 2672 | 250 | 1000 | <0.98 |
| *Enterobacter.* | 2643 | 250 | 1000 | 2000 |
| *Salmonella* sp. | 2482 | 250 | >1000 | 500 |
| *Serratia marc.* | 1814 | 31.2 | 125 | — |
| *Bacillaceae* | 1722 | 15.6 | 31.2 | <0.98 |

TABLE 3
Bactericide action at different time intervals of compounds $AB_5$ and $AB_6$ in comparison with chlorhexidine

| Microorganism | Compounds | | | | | | | | | | | | | | | | | |
|---|---|---|---|---|---|---|---|---|---|---|---|---|---|---|---|---|---|---|
| | $AB_5$ | | | | | | $AB_6$ | | | | | | Chlorhexidine** | | | | | |
| | (contact times min.) 3 | 5 | 10 | 30 | 60 | 120 | 3 | 5 | 10 | 30 | 60 | 120 | 3 | 5 | 10 | 30 | 60 | 120 |
| *E. coli* | – | – | – | – | – | – | + | + | – | – | – | – | + | + | + | – | – | – |
| Indole-positive *Proteus* | + | + | – | – | – | – | – | – | – | – | – | – | + | + | + | + | + | – |
| *Pseudomonas aeruginosa* | + | + | + | + | + | – | – | – | – | – | – | – | + | + | + | + | – | – |
| *Staph. aureus* | + | + | + | + | – | – | – | – | – | – | – | – | + | + | + | – | – | – |
| *B. subtilis* | – | – | – | – | – | – | – | – | – | – | – | – | + | + | + | + | – | – |
| *Serratia marcescens* | – | – | – | – | – | – | + | + | + | – | – | – | + | + | + | + | – | – |
| *Salmonella* sp. | + | + | – | – | – | – | – | – | – | – | – | – | + | + | + | + | – | – |

** 200 p.p.m.; +=growth –=no growth

TABLE 4

Interference of human serum (10%) on the bactericide activity

| Disinfectant | $AB_6$ | | | | | |
|---|---|---|---|---|---|---|
| Microorganism | Pseudomonas aeruginosa | | | | | |
| Contact time | 3 | 5 | 10 | 30 | 60 | 120 |
| Results: | | | | | | |
| without serum | – | – | – | – | – | – |
| with serum | + | + | + | + | – | – |

It is moreover noticed that the toxicity of compounds $AB_5$ and $AB_6$ is considerably low: the $LD_{50}$ in mice, by intraperitoneal route, has been determined as high as 85 mg/kg of body weight.

Finally, the irritability characteristics have been examined and can be summarized for both compounds as follows:

Cutaneous irritability (patch test)

The product, applied in powder (0.5 g) on the intact or scarified rabbit's skin, did not cause any irritative effect.

Ocular irritability (draize test)

The product, instilled as 5% suspension in the rabbits' conjunctival sac (0.1 ml), causes a slight ocular irritation only of the conjunctiva (vasal hyperhemia) without damages of cornea and iris.

The clinical picture regresses in the following 24—48 hours. The irritative phenomenon is inhibited by the water wash of the eye immediately after the instillation of the product.

**Claims**

1. Compositions having local antiseptic activity containing as the active principle N-haloamidine of formula (1):

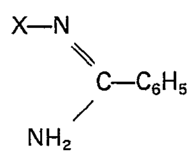

(1)

wherein X is a chlorine or a iodine atom.

2. Compositions with local antiseptic activity according to claim 1 in which the active principle is mixed with suitable solvents, diluents or inert excipients.

**Patentansprüche**

1. Zusammensetzungen mit antiseptischer lokaler Aktivität, die als Wirkstoff N-Amidinhalogenide der folgenden Formel (1):

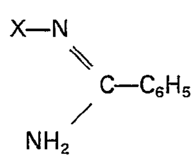

(1)

mit X=ein Chlor- oder Iod-atom enthalten.

2. Zusammensetzungen mit antiseptischer lokaler Aktivität nach Anspruch 1, wobei der Wirkstoff mit geeigneten Lösungsmitteln, Verdünnungsmitteln oder inert Zusatzmitteln gemischt wird.

**Revendications**

1. Compositions à activitè antiseptique topique contenant à titre de principe actif la N-haloamidine de formule (1):

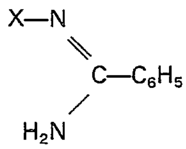

(1)

dans laquelle X est un atome de chlore ou de iode.

2. Compositions à activitè antiseptique topique selon la revendication 1, dans lesquelles le principe actif est melangé avec de solvants, diluants ou excipients inertes appropriés.